Europäisches Patentamt

European Patent Office

Office européen des brevets

(11) Publication number: **0 168 194**
A2

# (12) EUROPEAN PATENT APPLICATION

(21) Application number: 85304497.2

(22) Date of filing: 25.06.85

(51) Int. Cl.⁴: **A 61 M 1/32**

(30) Priority: 11.07.84 US 629692

(43) Date of publication of application:
15.01.86 Bulletin 86/3

(84) Designated Contracting States:
DE FR GB IT

(71) Applicant: AMERICAN HOSPITAL SUPPLY CORPORATION
One American Plaza
Evanston, IL 60201(US)

(72) Inventor: Viole, Anthony Joseph
209 A Fern Street
Newport Beach California 92663(US)

(74) Representative: Day, Jeremy John et al,
REDDIE & GROSE 16 Theobalds Road
London, WC1X 8PL(GB)

(54) Method and apparatus for oxygenating a crystalloid cardioplegia solution.

(57) A method of oxygenating a crystalloid cardioplegia solution comprising introducing a crystalloid cardioplegia solution into a container to provide a pool of the solution in the container, introducing oxygen gas directly into the pool of the solution in the container through a sparger whereby oxygen gas can flow upwardly through the solution in the container to oxygenate the solution, venting at least some of the oxygen gas from the container and withdrawing oxygenated solution from the container at a location below the sparger to minimize bubbles in the oxygenated solution.

FIG.2

Croydon Printing Company Ltd.

### METHOD AND APPARATUS FOR OXYGENATING A CRYSTALLOID
### CARDIOPLEGIA SOLUTION

During open heart surgery, the patient's heart is stopped and the blood is bypassed through an extracorporeal loop which pumps and oxygenates the blood. During this time, it is necessary to protect the heart from damage, and to do this, it is common practice to deliver a cold cardioplegia solution to the heart. The cardioplegia solution cools the heart, oxygenates the heart and may include various drugs beneficial to the heart.

Some cardioplegia solutions include blood. Those cardioplegia solutions that do not contain blood are known as crystalloid carioplegia solutions.

In order that a cardioplegia solution can oxygenate the heart, the solution itself must first be oxygenated. A conventional blood oxygenator can be used for this purpose. However, a typical blood oxygenator is relatively expensive and includes a defoamer to remove the foam from the oxygenated blood. Accordingly, there is a need for a less expensive apparatus which is particularly adapted for oxygenating crystalloid cardioplegia solutions.

This invention provides a method and an inexpensive apparatus for oxygenating crystalloid cardioplegia solutions. With this invention, the defoamer of the blood oxygenator is eliminated, and other important advantages are obtained.

With this invention, a crystalloid cardioplegia solution is introduced into a container to provide a pool of the solution in the container. Oxygen gas is introduced directly into the pool of the solution in the container through a sparger. The sparger is in the pool of the solution, and the oxygen gas can flow upwardly through the solution in the container to oxygenate the solution. By introducing the oxygen gas directly into the pool of the solution, less time is required to oxygenate the solution than if separately flowing streams of oxygen and cardioplegia solution were brought into contact outside of the pool. In addition, by introducing the oxygen gas into the pool, oxygenation can occur whether or not the solution in the pool is being recirculated.

To prevent pressurizing of the container, at least some of the oxygen gas is vented from the container above the upper level of the solution in the container. The oxygenated solution is drawn from the container through a port at a location below the sparger. By withdrawing the oxygenated solution at this location, the number of bubbles in the withdrawn solution is minimized.

The vertical separation between the withdrawal location and the sparger should be sufficient so as to produce this effect. Of course, by increasing this spacing, the bubbles in the withdrawn solution tend to be reduced. By way of example, a vertical spacing of about one inch between the bottom of the sparger and the central axis of the port in the container through which the oxygenated solution is withdrawn is generally sufficient.

Preferably, the solution is introduced into the container through a port which is also below the sparger. In this event, either of the two above-mentioned ports, which are used for solution recirculation, can be used for the inlet or the outlet. This facilitates connection of the container into the cardioplegia system in that it is much more difficult for the technician to incorrectly couple the container into the cardioplegia system.

Although the oxygen gas may be introduced in different ways, it is preferred to introduce the gas through an upwardly extending tube in the container. With this construction, the sparger is carried by the tube.

In a preferred construction, the container has a bottom wall, and both of the ports for solution recirculation are located in the bottom wall. The tube is preferably carried by the bottom wall and projects above it so that the sparger is sufficiently above the two ports.

The invention will now be described in greater detail by way of example with reference to the drawings, in which

Fig. 1 is a fragmentary, partially schematic isometric view of an apparatus for oxygenating a crystalloid cardioplegia solution.

Fig. 2 is an elevational view of the container.

Figs. 3-5 are enlarged sectional views taken generally along lines 3-3, 4-4 and 5-5, respectively, of Fig. 2.

Fig. 6 is a fragmentary view taken generally along line 6-6 of Fig. 5.

Fig. 7 is a sectional view taken on an axial plane through the sparger.

Fig. 8 is an enlarged, fragmentary sectional view illustrating one of the ports in the sparger.

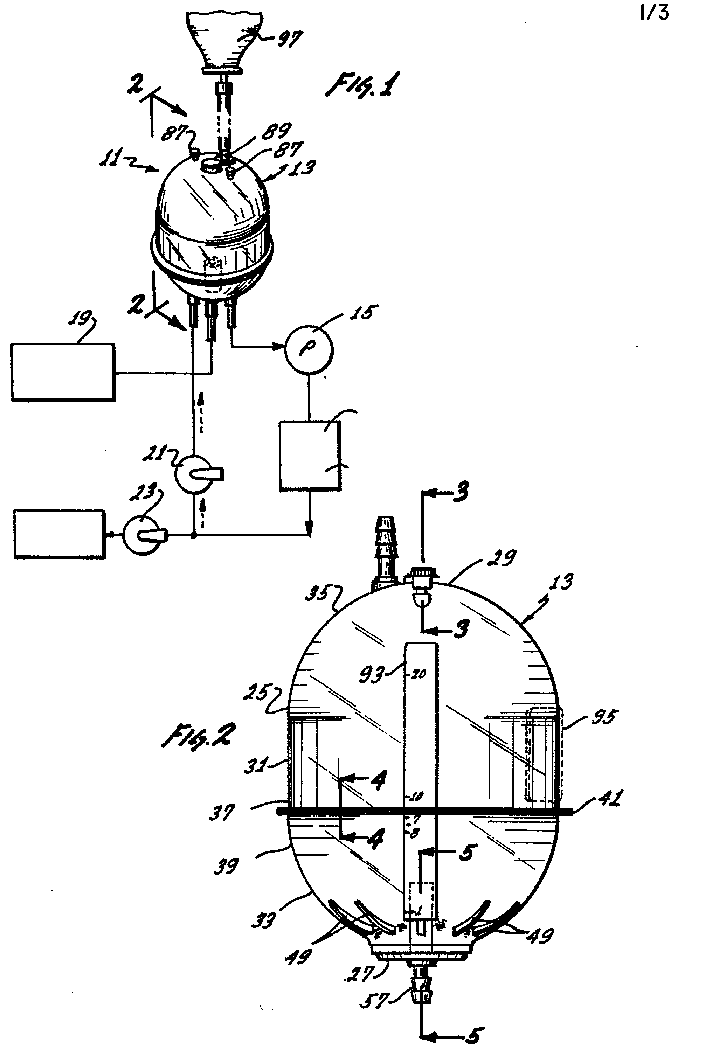

Fig. 1 shows an apparatus 11 for oxygenating a crystalloid cardioplegia solution which generally includes a container 13, a pump 15, a heat exchanger 17, an oxygen gas supply 19 and manual valves 21 and 23. The apparatus 11 also includes flexible tubing for interconnecting the components of the apparatus as shown in Fig. 1. Except for the container 13, the apparatus 11 may be conventional.

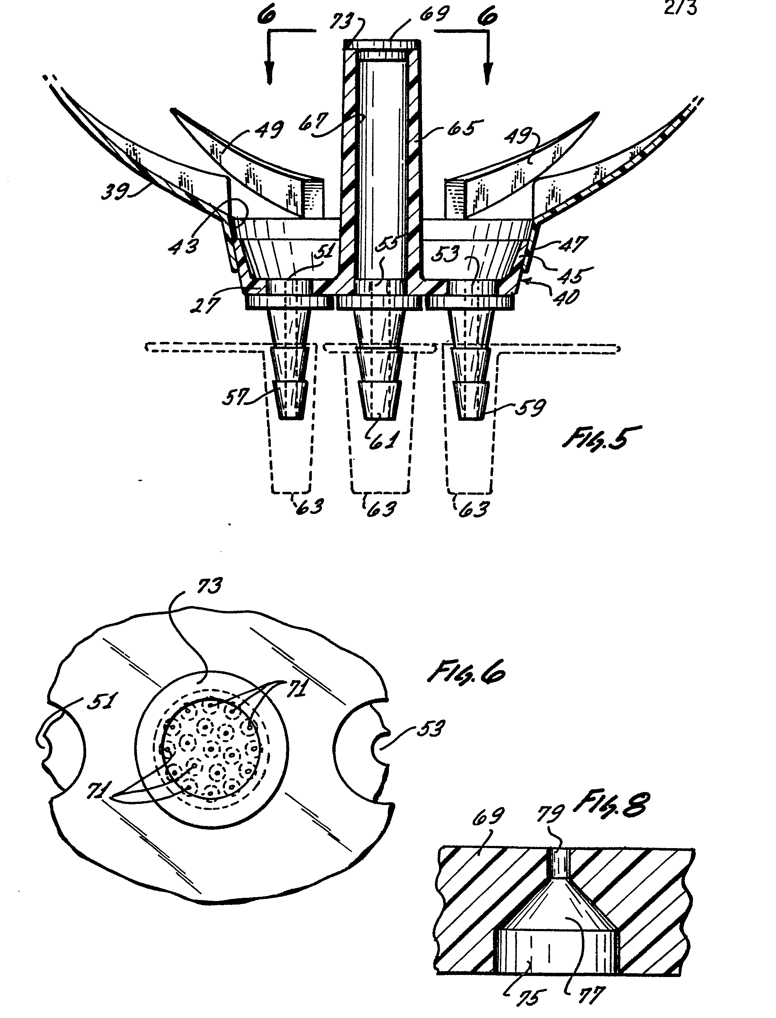

The container 13 has a peripheral wall 25 (Fig. 2), a bottom wall 27 and a top wall 29, with the bottom and top walls also forming end walls of the container. Although various constructions are possible, in the embodiment illustrated, the walls 25, 27 and 29 are each constructed of a suitable transparent plastic material, and the peripheral wall 25 has a cylindrical section 31 and part-spherical end sections 33 and 35.

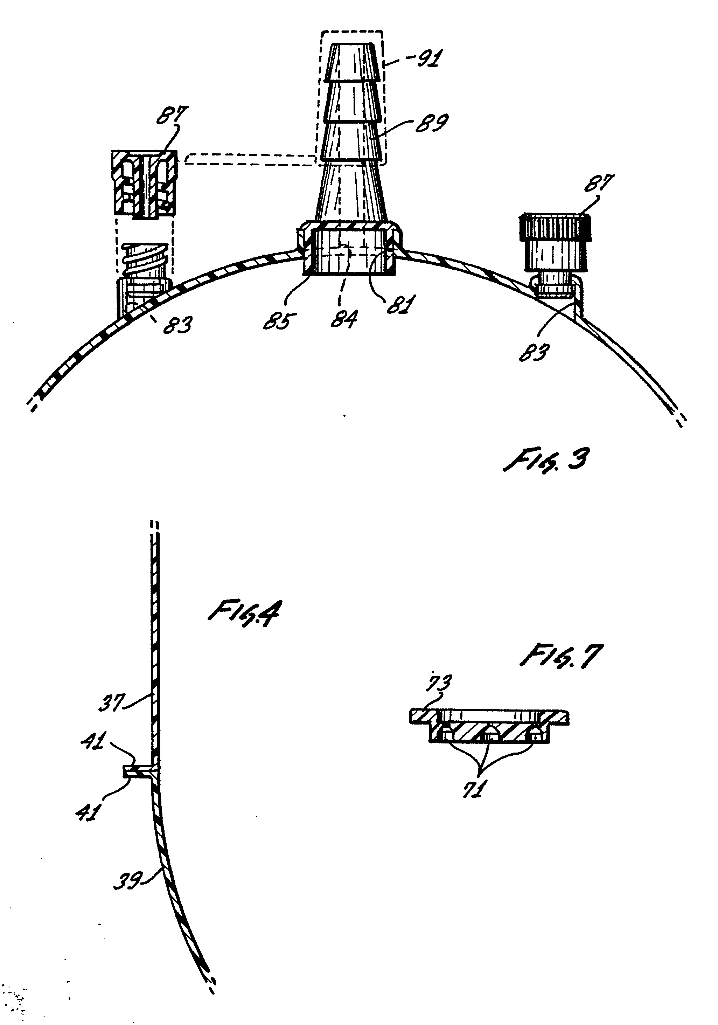

Although the container 13 may be formed from any suitable number of components, in the illustrated embodiment, it includes container sections 37 and 39 and a bottom wall section 40 (Fig. 5). The container sections 37 and 39 have flanges 41 which are adhered to each other as shown in Figs. 2 and 4. The container section 37 defines the cylindrical section 31 and the end section 35, and the container section 39 defines the end section 33.

The end sections 33 and 35 are generally hemispherical, and the end section 33 has an axial, circular opening 43 (Fig. 5). The bottom wall section 40 has a peripheral flange 45 which extends around the bottom wall 27 and which is adhered to a flange 47 of the container section 39. The lower end of the wall section 39 has ribs 49 extending along its interior surface toward the bottom wall 27 as shown in Figs. 2 and 5.

The bottom wall 27 has an inlet port 51, an outlet port 53 and an oxygen gas port 55 intermediate the inlet port and the outlet port as shown in Fig. 5. The bottom wall section 40 includes an inlet fitting 57, an outlet fitting 59 and an oxygen gas fitting 61 in the form of male tubing connectors are formed integrally with the bottom wall 27 and in axial communication with the ports 51, 53 and 55, respectively. Prior to being coupled into the remainder of the apparatus 11, the fittings 57, 59 and 61 can be suitably closed by removable caps 63 as shown in dashed lines in Fig. 5.

The bottom wall section 40 includes a tube 65 formed integrally with the bottom wall 27 in axial alignment with the oxygen gas port 55. The tube 65 projects axially upwardly above the bottom wall 27 and above the ports 51 and 53. Although various constructions are possible, the tube

6

0168194

65 is cylindrical and has a cylindrical interior passage 67 (Fig. 5).

A gas sparger 69 is carried by the tube 65 at the upper end of the tube. The sparger 69, which may also be constructed of clear, transparent plastic, is generally in the form of a plate having numerous orifices 71 extending through it and a peripheral flange 73. The orifices 71 can be arranged in any suitable pattern and be of various different configurations. However, preferably, the orifices 71 each have a relatively large diameter inlet section 75, a tapered intermediate section 77, and a small-diameter outlet section 79 as shown in Fig. 8. In the form shown in Fig. 8, the sections 75, 77 and 79 are coaxial, the sections 75 and 79 are cylindrical and the section 77 is conical. The sparger 69 is adhesively attached to the upper end of the tube 65 with the flange 73 resting on the upper end of the tube and with a portion of the sparger 69 extending slightly into the passage 67. Thus, the sparger 69 forms a perforated end wall for the tube 65.

A suitable number of ports may be formed in the peripheral wall 25 and/or the top wall 29, and in the embodiment illustrated, a central port 81, two ports 83 and a priming port 84 are formed in the top wall 29 as shown in Figs. 1-3. The central port 81 is permanently closed with a cap 85 and not used, and the two ports 83 are closed with threaded caps 87. The port 84 is aligned with a male tubing fitting 89, and the fitting may be closed with a cap 91 as shown in dashed lines in Fig. 3. The port 83 may be used for adding medication to the interior of the container 13 or for venting the container. The ports 83 and 84 are well above the sparger 69. The exterior of the container 13 may have graduations 93 to show the level of the solution therein, and a label 95 with identifying information may also be placed on the exterior of the container.

In use of the apparatus 11, a crystalloid cardioplegia solution is introduced from a suitable source, such as a bottle 97, into the container 13 through the priming port 84 to provide a pool of the solution in the container. Although the level of the pool of the solution in the container can vary, it is preferably well above the sparger 69. Preferably, the vertical spacing between the top of the sparger 69 and the ports 51 and 53 is at least one inch. The upper level of the pool is below the ports 83. Oxygen gas is introduced from the supply 19 directly into the pool of the solution in the container 13 through the oxygen gas port 55, the tube 65 and the sparger 69. The sparger 69 is in the pool of the solution in the container 13 so that the oxygen gas can flow upwardly through the solution in the container to oxygenate the solution. To prevent pressurizing of the container 13, the oxygen gas is vented from the container above the upper level of the pool of solution in the container through one of the ports 83, and if desired, a medication can be introduced through the other of the ports 83. The oxygenated solution is withdrawn from the container through the outlet port 53 by the pump 15. Because the location of the outlet port 53 is below the sparger 69, there are a minimum of oxygen bubbles in the oxygenated solution. The solution is then pumped through a heat exchanger 17 where it is cooled and delivered in its cooled condition to the valves 21 and 23. If it is desired to recirculate the solution, the valve 23 is closed, and the valve 21 is open. Alternatively, to deliver the oxygenated solution to a patient, the valve 21 is closed and the valve 23 is open.

Because both of the ports 51 and 53 are well below the sparger 69, the crystalloid cardioplegia solution is introduced into the container 13 at a location which is well below the sparger 69. In addition, the inlet port 51 and the outlet port 53 can be used for the outlet and inlet, respectively, if desired, thereby making incorrect hook-up of the container 13 into the system much less likely to occur. Also, by positioning the oxygen gas port 55 intermediate the inlet port 51 and the outlet port 53, the recirculating cardioplegia solution is forced to pass over the sparger to receive the oxygen gas.

The ports 51 and 53 are preferably in the bottom wall 27, although they may be placed in the peripheral wall 25 closely adjacent the bottom wall 27. The tube 65 preferably extends upwardly, and the sparger 69 preferably faces upwardly in the pool of the solution in the container.

CLAIMS

1.   A method of oxygenating a crystalloid cardioplegia solution comprising:

introducing a crystalloid cardioplegia solution into a container to provide a pool of the solution in the container;

introducing oxygen gas directly into the pool of the solution in the container through a sparger with the sparger being in the pool of the solution whereby the oxygen gas can flow upwardly through the solution in the container to oxygenate the solution;

venting at least some of the oxygen gas from the container above the upper level of the pool of the solution in the container; and

withdrawing oxygenated solution from the container at a location below the sparger to minimize oxygen bubbles in the oxygenated solution.

2.   A method according to claim 1 wherein the crystalloid cardioplegia solution is introduced into the container at a location which is below the sparger.

3.   A method according to claim 1 or claim 2 wherein said introduction and withdrawal locations are closely adjacent the bottom wall of the container.

4.   A method according to claim 3 wherein the oxygen gas is introduced at a location between said introduction and withdrawal locations.

5.    A method according to any preceding claim
wherein the oxygen gas is introduced upwardly through the
sparger into the pool.

6.    A method according to any preceding claim
wherein the oxygen gas is introduced through an upwardly
extending tube in the container and within the pool with
the sparger being carried by the tube.

7.    Apparatus for oxygenating a crystalloid
cardioplegia solution comprising :

   a container;
means for introducing a crystalloid cardioplegia
solution into the container to provide a pool of the solution
in the container;

   a sparger within said container, said sparger being
adapted to be below the upper level of the pool in the
container;

   means for introducing oxygen gas directly into the
pool of the solution in the container through said sparger
whereby the oxygen gas can flow upwardly through the solution
in the container to oxygenate the solution;

   means for venting at least some of the oxygen gas
from the container above the upper level of the solution in
the container; and

means for withdrawing oxygenated solution from the container at a location below the sparger to minimize oxygen bubbles in the oxygenated solution.

8. Apparatus according to claim 7 wherein said introducing means is located to introduce the solution into the container at a location which is below the sparger.

9. Apparatus according to claim 7 or claim 8 wherein said location is closely adjacent the bottom wall of the container.

10. Apparatus according to any one of claims 7 to 9 wherein said oxygen gas introducing means includes a tube mounted on said container and extending within the container, at least a portion of said tube extending upwardly and said sparger is carried by said upwardly extending portion of said tube.

11. Apparatus for oxygenating a crystalloid cardioplegia solution comprising:
a container having a peripheral wall and a bottom wall joined to the peripheral wall;
said bottom wall having an inlet port and an outlet port through which the crystalloid cardioplegia solution can be supplied to and removed from, respectively, the container;
a tube mounted on said bottom wall and projecting upwardly above said bottom wall and said ports, said bottom wall having a oxygen gas port communicating with the tube whereby oxygen gas can be supplied to said tube;

a sparger carried by said tube adjacent the upper end thereof, said sparger being above said ports and said bottom wall; and

a vent opening in said container substantially above said sparger.

Fig.1

Fig.2

Fig.5

Fig.6

Fig.8

FIG. 3

FIG.4

FIG.7